# EUROPEAN PATENT SPECIFICATION

(11) **EP 2 374 004 B1**
(45) Date of publication and mention of the grant of the patent: **05.12.2012**
(21) Application number: 09771643.5
(22) Date of filing: 16.12.2009
(51) Int. Cl.: G01N 33/68, H01J 49/16

(54) **RECOMBINANT VON WILLEBRAND FACTOR AS A MOLECULAR WEIGHT MARKER FOR MASS SPECTROMETRY ANALYSES**
REKOMBINANTER VON-WILLEBRAND-FAKTOR ALS MOLEKULARGEWICHTSMARKER FÜR MASSENSPEKTROMETRISCHE ANALYSEN
FACTEUR DE VON WILLEBRAND RECOMBINANT UTILISÉ COMME MARQUEUR DE POIDS MOLÉCULAIRE DANS DES ANALYSES PAR SPECTROMÉTRIE DE MASSE

(30) Priority: 23.12.2008 US 140475 P
(43) Date of publication of application: 12.10.2011
(73) Proprietor: Baxter International Inc., Deerfield, IL 60015 (US); Baxter Healthcare SA, 8152 Glattpark (Opfikon) (CH); Technische Universität Wien, 1040 Wien (AT)
(72) Inventor: KEMPTNER, Jasmin, A-4663 Laakirchen (AT); MARCHETTI-DESCHMANN, Martina, A-1230 Vienna (AT); MUELLER, Roland, A-2500 Baden (AT); IVENS, Andreas, Zürich 8049 (CH); TURECEK, Peter, A-3400 Klossterneuburg (AT); SCHWARZ, Hans-Peter, A-1180 Vienna (AT); ALLMAIER, Guenter, A-1020 Vienna (AT)
(74) Representative: Bassett, Richard Simon
(86) International application number: PCT/US2009/068258
(87) International publication number: WO 2010/075132

(56) References cited:
- WO-A1-00/49047
- WO-A2-2005/114220
- WENZEL R J ET AL: "Analysis of megadalton ions using cryodetection MALDI time-of-flight mass spectrometry" ANALYTICAL CHEMISTRY 20050715 US, vol. 77, no. 14, 15 July 2005 (2005-07-15) , pages 4329-4337, XP002569231 ISSN: 0003-2700
- ZAIA JOSEPH ET AL: "Structural analysis of cartilage proteoglycans and glycoproteins using matrix-assisted laser desorption/ionization time-of-flight mass spectrometry" ANALYTICAL BIOCHEMISTRY, ACADEMIC PRESS INC, NEW YORK, vol. 277, no. 1, 1 January 2000 (2000-01-01), pages 94-103, XP002373490 ISSN: 0003-2697

## Description

### CROSS REFERENCE TO RELATED APPLICATIONS

### BACKGROUND

Von Willebrand factor (VWF) is an adhesive complex glycoprotein with a molecular mass of the monomer of about 260 kDa (determined by SDS agarose gel electrophoresis¹⁻³), VWF circulating in human plasma as a dimer and oligomers ranging in molecular mass from 450 kDa to 20 000 kDa^{2,4,5}. The precursor polypeptide, pre-pro-VWF, synthesized in endothelial cells^{6,7} and megakaryocytes⁸, consists of a 22-amino acid residue signal peptide, a 741-residue pro-peptide and a 2050-residue polypeptide⁹. After *in vivo* removal of the signal peptide two pro-VWF units are linked via disulfide bonds forming dimers, the building blocks for mature VWF multimers^{10,11}. Prior to plasma release the pro-peptide is cleaved off and the remaining part contains several post translational modifications, namely glycosylation^{9,12-14} and sulfation¹⁵. The final mature VWF carries 12 N- and 10 O-glycans, whereof specific N-glycans are further modified by sulfation¹⁶.

The carbohydrate moiety contributes to several specific properties of VWF, e.g. physiological activity, receptor binding, clearance from plasma circulation and structural stability^{17,18}. After biosynthesis, mature VWF is either released into the blood circulation system or it is stored in the Weibel-Palade body¹⁹. In human plasma, VWF serves a dual purpose in hemostasis. In case of vascular injury, it mediates adhesion of platelet glycoproteins, e.g. collagen to vascular subendothelium²⁰, Furthermore, VWF builds a non-covalent complex with blood coagulation factor VIII (FVIII) avoiding its rapid clearance from plasma, thus normal thrombin generation can take place²¹.

Inherited defects on VWF cause von Willebrand Disease (VWD), one of the most common bleeding disorders in humans²². Symptoms of VWD are abnormal platelet function leading to constant nose bleeding, unexplained bruising or prolonged bleeding after injury. Due to the reduced level of VWF, VWD is commonly accompanied by a decrease of FVIII procoagulant activity. Depending on the severity of VWF defect, several degrees of VWD occur. Patients with mild VWD have reduced VWF plasma levels and are treated by administration of desmopressin, which temporarily increases the VWF level²³. Patients suffering from more severe forms of VWD have to be treated with human plasma derived concentrates or cryoprecipitates, containing both FVIII and VWF^{24,25}. However, this replacement therapy is accompanied by some limitations²⁶, Human plasma derived VWF concentrates show varying VWF and FVIII levels and ratios, depending on the donor's pool. Furthermore, due to proteolytic degradation during the isolation and purification (manufacturing) process, the multimer composition of the plasma derived VWF concentrates varies and no high molecular mass multimers, exhibiting the highest hemostatical activity, can be found. The use of recombinant VWF (rVWF) produced by fermentation of transformed cells can overcome these limitations²⁷. Absence of plasma proteases avoids rVWF degradation and the risk of virus transmission is nearly eliminated. *In vivo* and *in vitro* evaluations have confirmed that structure and properties of rVWF are comparable to plasma derived VWF²⁸.

Molecular weight determination of analytes using mass spectrometry is limited by the availability of standards, i.e., molecules of known exact molecular weight and well-defined composition from batch-to-batch at reasonable costs that can be used to correlate an unknown mass of an analyte to the known mass of the standard. If an analyte has a mass that is significantly outside a range about the mass of a known standard, the accuracy of the correlation is decreased. To date, well-defined protein/glycoprotein standards for use in mass spectrometry analysis of analytes having a molecular weight greater than 100 kDa are limited. Immunoglobulin proteins have been used, but their masses are limited to suitability of analytes having a molecular weight up to about 150 kDa. Thus, a need exists for a molecular weight standard that can be used to determine the molecular weight of analytes having masses greater than, e.g., 200 kDa.

WO 2005/114220 discloses protein-containing compositions for the calibration of mass spectrometers. Wenzel et al (2005) Anal. Chem. 77, 4329-4337 discloses the analysis of megadalton ions, such as vWF, using mass spectrometry.

### SUMMARY

Disclosed herein is a method of determining the molecular weight of an analyte using mass spectrometry (MS) by measuring the mass spectrum of the analyte and correlating it to the mass spectrum of a recombinant VWF (rVWF) of known molecular weight. The mass spectrum of the rVWF can be obtained by measuring it directly or by referring to a previously prepared spectrum. The analytes being analyzed in the disclosed methods are those having a high molecular weight (MW; the term molecular mass can be used synonymously), e.g., at least 200 kDa. In some embodiments, the analyte has a MW of at least 1000 kDa or at least 1500 kDa. In specific embodiments, the analyte is an antibody or another large protein such as fibrinogen or collagen or derivatives thereof

In some cases, the method disclosed further comprises calculating the MW of the VWF molecular weight marker by determining the number of monomers of VWF (SEQ ID NO: 1) in VWF molecular weight marker. In various embodiments, the sample and the VWF molecular weight marker are desalted prior to analysis by MS.

### BRIEF DESCRIPTION OF THE FIGURES

Figure 1 shows CGE-on-the-chip electropherograms of rVWF samples A, B and C under reducing conditions without pre-purification. (FU, fluorescence units, s, seconds in migration time).

Figure 2 shows an SDS-PAGE image of rVWF sample A under reducing conditions after various desalting procedures.

Figure 3 shows nano ES GEMMA spectra of rVWF samples A, B and C after reduction of disulfide bridges and desalting with MicroSpin™ devices.

Figure 4 shows positive ion MALDI mass spectra obtained in the linear mode of rVWF samples A, B and C. M indicates mature rVWF and P indicates pro-rVWF.

### DETAILED DESCRIPTION

As described herein, monomers and multimers of rVWF are used as mass spectrometry molecular weight markers for analytes of high MW because the MWs of multimeric rVWF is easily be calculated based upon the known MW of the repeating rVWF monomers (SEQ ID NO: 1). Thus, knowing the MW of the VWF multimer allows its use to calibrate (i.e., a calibrant) mass spectrometry techniques use to determine the mass of an analyte of interest. The MW of rVWF includes the protein moiety and the glycan moiety of glycosylated rVWF if it is used from a source that produces reproducibly glycosylated VWF.

Due to the ability to prepare high molecular mass rVWF multimers (based upon the number of monomers of VWF), it was discovered that rVWF is useful as a molecular weight marker for mass spectrometry analyses of analytes having high (e.g., greater than 200 kDa) molecular weights. The molecular weight of multimers of VWF is calculated based upon the number of repeating units of the VWF monomer (SEQ ID NO: 1). Depending upon the nature of the analyte to be assessed, a rVWF multimer is prepared having the appropriate mass to be a suitable marker for the analyte of interest, anywhere from 200 kDa to over 5,000 kDa. For example, when an analyte is suspected of having a mass in a specific range, a monomer and one or more ic and/or multimeric forms of rVWF are prepared that have a sufficient number of repeating rVWF monomers to provide a mass within, for example and without limitation, about 10 kDa of the suspected (or expected) mass of the analyte. Then, the spectrum of the analyte is compared to the spectrum of the rVWF marker or markers to provide a mass for the analyte. One benefit of using rVWF as a standard for mass spectrometry analysis is that the rVWF standard used does not have to contain only one multimer. Due to the repeating mass units of the rVWF monomers, even a mixture of different multimers is useful because their masses are evenly spaced by the mass of the VWF monomer or dimer.

The monomer, dimer and multimers of rVWF are typically connected using disulfide bonds.

VWF can be used as a standard, alternatively referred to herein as a molecular weight marker, for analytes having a molecular weight of at least 200 kDa, at least 250 kDa, at least 300 kDa, at least 350 kDa, at least 400 kDa, at least 450 kDa, at least 500 kDa, at least 550 kDa, at least 600 kDa, at least 650 kDa, at least 700 kDa, at least 750 kDa, at least 800 kDa, at least 850 kDa, at least 900 kDa, at least 950 kDa, at least 1000 kDa, at least 1100 kDa, at least 1200 kDa, at least 1250 kDa, at least 1300 kDa, at least 1350 kDa, at least 1400 kDa, at least 1450 kDa, at least 1500 kDa, or at least 2000 kDa.

Preparation of a rVWF having a desired molecular weight can be achieved by expression from mammalian cells and had been described in the literature (Fischer, Thromb Thrombolysis, 8(3):197-205, 1999, Schwarz, et al., Semin Thromb Hemost,28(2):215-26, 2002, Turecek, et al., Histochem Cell Biol., 117:123-129, 2002, Cytotechnology. 30(1-3):1-16, 1999)

Nano ES (electrospray) gas phase electrophoretic mobility macromolecular analyzer (GEMMA), a particular kind of ion mobility spectrometer, vacuum matrix-assisted laser desorption ionization (MALDI) mass spectrometry (MS) and additionally capillary gel electrophoresis-on-the-chip (CGE-on-the-chip) can be used to determine the molecular weight of an analyte. Contrary to the commonly used approach, SDS-polyacrylamide²⁹ and -agarose gel electrophoresis^{3o}, determination of the exact molecular size, sample heterogeneity and exact molecular mass of three rVWF preparations was performed by nano ES GEMMA, MALDI-linear TOF (time-of-flight) MS as well as CGE-on-the-chip.

Nano ES GEMMA analysis is based on generating multiply charged molecular ions by means of a nano ES process in the cone-jet mode followed by charge reduction via polonium-210, thus yielding neutral and singly charged positive and negative ions. These ions are size separated according to their electrophoretic mobility diameter (EMD) using a nano differential mobility analyzer (nDMA) and detected by means of a condensation particle counter (uCPC) based on an indirect optical detection³¹⁻³³. Based on the correlation (r = 0.998) of the EMD to the molecular mass of well defined globular proteins, the molecular mass of glycoproteins can be determined³⁴. GEMMA analysis is generally discussed in Allmaier, et al., J. Mass. Spec., 36:1038-1052 (2001).

The second method used for characterization of rVWF was positive ion MALDI MS applying a linear TOF system with a standard detector³⁵. The well-established "soft" desorption/ionization technique MALDI generating mainly singly to triply charged molecular ions, showing a high salt and detergent tolerance and requiring low amounts of samples, makes MALDI TOF MS to the dedicated technique for accurate molecular mass determination of intact high mass proteins³⁶. Discussion of MALDI can be found in U.S. Patent No. 7,351,959, incorporated herein by reference.

### EXAMPLES

### Chemicals

Ammonium acetate p.a., acetic acid 96 %, acetonitrile p.a. (ACN), methanol p.a. (MeOH), formic acid 98 - 100 % (FA) and water p.a. (conductivity at 25 °C ≤ 1 µS/cm) were purchased from Merck (Darmstadt, Germany). Coomassie Brilliant Blue R250 (CBB), sodium dodecyl sulphate (SDS) and 2,4,6 trihydroxyacetophenone monohydrate (THAP) were obtained from Fluka (Buchs, Switzerland). Lithium dodecyl sulphate (LDS) and 1,4-dithiothreitol (DTT, min 99.0 %) were obtained from Sigma-Aldrich (Steinheim, Germany). NuPage LDS sample buffer (4×), NuPage Tris-Acetate SDS running buffer (20×), HiMark unstained high molecular weight protein standard and Invitromass high molecular weight mass calibration kit were purchased from Invitrogen (Carlsbad, CA, USA). Trifluoroacetic acid (TFA) was obtained from Riedel de Haen (Seelze, Germany).

### Recombinant von Willebrand factor (rVWF)

Three non-commercial rVWF preparations were produced from Baxter Biosciences (Vienna, Austria). Sample A contained 0.67 mg/mL rVWF dissolved in a buffer system consisting of 5.25 g/L L-glycerin, 5.25 g/L L-lysine-hydrochloride, 5.25 g/L trisodium-citrate × 2H₂O, 0.62 g/L CaCl₂ × 2H₂O and 2.2 g/L NaCl (pH 6.8). Sample B contained 0.49 mg/mL rVWF and sample C 0.54 mg/mL rVWF; both samples were dissolved in the following buffer system consisting of 20 mM HEPES, 150 mM NaCl and 0.5 % saccharose (pH 7.4).

### Capillary gel electrouhoresis-on-the-chip for evaluation of sample homogeneity

Prior to detailed characterization via nano ES GEMMA and MALDI TOF MS, the rVWF samples were investigated with CGE-on-the-chip, yielding a first overview of the three samples in terms of homogeneity and approximate molecular mass within a reasonable time span (60 seconds of separation time).

CGE-on-the-chip experiments were carried out using the 2100 Bioanalyzer and the Protein P200 plus assay (Agilent Technologies, Waldbronn, Germany) with a non-commercial set-up. The instrument was operated and the obtained results were integrated and evaluated with the aid of the 2100 Expert software v.B.02.02.SI238.

All chips (Agilent Technologies, Waldbronn, Germany) were prepared according to the manufacturer's instruction. Briefly, 4 µL of the rVWF sample solutions were mixed with 2 µL of the provided denaturing solution containing 0.0052 mg/mL DTT, heated on 95 °C for 5 min and finally the mixture was diluted with 84 µL water. The final sample solution (6 µL) was applied into the designated sample wells on the CGE-chip and electrokinetically injected. For molecular mass determination a protein ladder consisting of well-defined proteins (provided in the Agilent Technologies kit) was treated in the same way.

### SDS-PAGE for salt/deterpent removal optimization

For analysis of protein samples under reducing conditions 6 µL sample solution, 3 µL NuPage LDS sample buffer (4×) and 3 µL 0.1 M DTT were mixed, incubated for 1 min at 99 °C and cooled to room temperature. The resulting mixture (10 µL) was applied directly onto the slab gel. For molecular mass estimation the HiMark protein standard mixture was applied onto the gel additionally. A mixture containing 6 µL HiMark protein standard, 3 µL LDS sample buffer (4×) and 3 µL water were directly applied onto the gel. Electrophoresis was performed on a 3-8 % Tris-Acetate, 1mm × 10 wells NuPage precast mini gel (Invitrogen, Carlsbad, CA, USA) using freshly prepared Tris-Acetate SDS running buffer. Constant voltage was set to 150 V, after 70 min the separation was stopped. Directly after the separation the gel was placed in a fixing solution (45 % MeOH, 5 % acetic acid) for 30 min and subsequently stained with a Coomassie staining solution (0.1 % CBB R250, 45 % MeOH, 9 % acetic acid) for 45 min. Finally, the gel was placed in a destaining solution (45 % MeOH, 9 % acetic acid) for 45 min, within this time frame the gel background became completely clear and distinct protein bands became visible.

### Sample preparation methods for nano ES GEMMA analysis

Due to the differing buffer compositions containing a high amount of salts, additives and detergents, the three rVWF samples were desalted/purified prior to application to the nano ES GEMMA device. Three different desalting approaches were evaluated, membrane centrifugation, size exclusion chromatography and dialysis. rVWF loss was monitored during these desalting/purifying procedure by SDS-PAGE.

For membrane centrifugation Nanosep™ centrifugal devices with a MW cut off of 3000 Da (Pall Corporation, Ann Arbor, MI, USA) were used. rVWF sample solution (300 µL) was spun down 20 min at 5000 rpm, the residue was washed once with 20 mM ammonium acetate buffer (100 µL, pH 6.8) and re-suspended in 20 mM ammonium acetate buffer (300 µL, pH 6.8).

Size exclusion chromatography was performed with MicroSpin™ G-25 columns (GE Healthcare, Little Chalfont, UK). Due to the limitations of the MicroSpin™ column, the sample was split into two aliquots and desalted separately, twice for 2 min at 2000 rpm, each time using a new spin column. The aliquots were then recombined for GEMMA analysis.

Dialysis of rVWF preparations was performed in Slide-A-Lyzer® dialysis cassettes with a MW cut off of 10 kDa (Pierce, Rockford, IL, USA). rVWF solution (300 µL) was diluted to a volume of 1.5 mL and dialyzed approximately 24 h in a dialysis solution (20 mM ammonium acetate, pH 6.8).

The rVWF samples were then reduced to break the disulfide bonds by mixing with 2 % (w/v) SDS and 0.15 % (w/v) DTT, incubation for 1 min at 99 °C. The resulting reduced rVWF was then analyzed by GEMMA analysis.

### Nano ES GEMMA

The nano ES GEMMA system (TSI 3980) consists of a nano ES charge reduction unit (TSI 3480), a nano differential mobility analyzer (nDMA; TSI 3080) and an ultrafine condensation particle counter (µCPC; TSI 3025A) as detector (all parts from TSI Inc, Shoreview, MN, USA). Multiply charged ions were generated by the nano electrospray unit and charge reduced by a bipolar atmosphere generated by means of polonium-210 to yield neutral and singly charged species. These species were size separated according to their electrophoretic mobility diameter (EMD) within a flow of particle-free air in the nDMA and detected with the µCPC.

For molecular mass determination of rVWF, the EMD of several well defined standard proteins were determined and correlated with their molecular mass. Based on the resulting relationship between EMD and molecular mass, the size of unknown molecules as the rVWF of this study were determined to provide the molecular mass^{34,37}.

The nano ES source was set at 2 kV spray voltage and 0.3 L/min CO₂ (99.995 %, Air Liquide, Schwechat, Austria). Compressed air (generated by compressor type Hobby-Star 200W, AGRE, Garsten-St. Ulrich, Austria, at 1 L/min) was applied to the instrument. A fused silica capillary with an inner diameter of 150 nm was used, and the tip of the spray needle was positioned at an angle of 75°. The ES ionization was operated in the positive ion mode. Ten to twenty scans (120 s per scan) across the whole size range were averaged for each final nano ES GEMMA spectrum presented herein.

### Sample preparation for MALDI mass spectrometry

For reduction of the disulfide bonds in the rVWF dimers and multimers, a VWF solution (10 µL)was mixed with 10 % LDS (w/v, 0.5 µL) and 1 M DTT (1 µL). The resulting mixture was vortexed thoroughly, spun down and incubated at 99 °C for 1 min. Afterwards, sample desalting/purification was performed with ZipTip™ pipette tips containing a hydrophilic HPL resin (Millipore, Bedford, MA, USA). Then, 10 µL solution A (ACN/ 0.1 % FA, 9:1, v/v) was mixed with the sample solution. For wetting the HPL stationary phase, 10 µL of solution B (ACN, 0.1 % FA, 1:1, v/v) was aspirated and dispensed to waste. Equilibration of the resin was obtained by aspirating and dispensing solution A three times. The sample was bound to the resin by aspirating and dispensing the sample solution seven times. To remove all interfering salts, additives and detergents the tip was washed with plain solution A ten times. Then, the VWF was eluted with 2 to 4 µL MALDI matrix solution (10 mg THAP dissolved in 1 mL 0.1 % TFA/ACN (1:1, v/v) solution). For MALDI MS analysis 0.5 µL of the resulting sample/matrix solution were applied onto the stainless steel MALDI target. Calibration was performed externally by using the Invitromass high molecular weight mass calibration kit. The calibration kit was applied according to the manufacturer's instructions.

### MALDI Mass Spectrometry

All MALDI TOF MS experiments were carried out on an AXIMA TOF² (Shimadzu Biotech Kratos Analytical, Manchester, UK) equipped with a nitrogen laser (λ = 337 nm). The instrument was operated in positive ion, linear mode without using pulsed extraction. Each mass spectrum was acquired by averaging 50 to 200 unselected and consecutive laser shots. Prior to data analysis the mass spectra were smoothed with Savitsky-Golay algorithm.

### Results

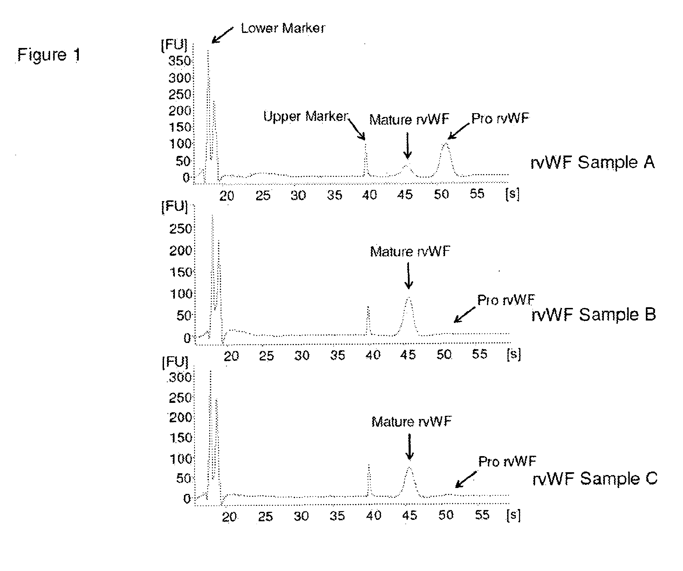

The homogeneity of the rVWF samples was measured using CGE-on-the-chip. Without any particular sample preparation and within a short time span (60 sec run time) data of rVWF samples A, B and C (Figure 1) were obtained, revealing that each sample had two peaks corresponding to two different rVWF species. They were determined to be mature rVWF, having a molecular mass of 277 kDa, and pro-rVWF, having a molecular mass of 341 kDa. The presence of the pro-rVWF was rationalized as due to incomplete enzymatic cleavage to the mature rVWF. The major component of Sample A was pro-rVWF with some mature rVWF (see Figure 1, top). The analyses of Samples B and C showed that mature rVWF was the major component, with little pro-rVWF.

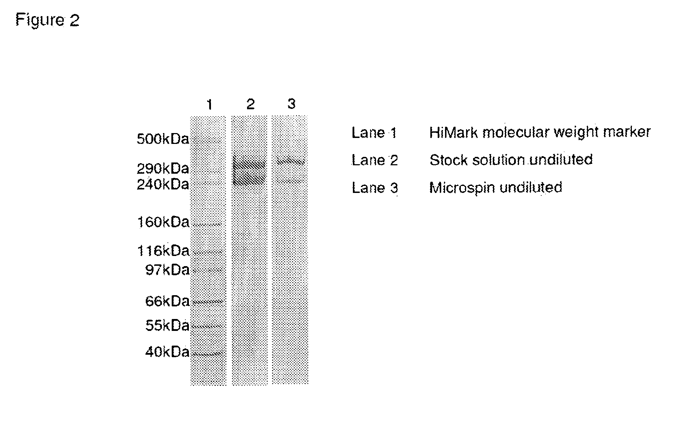

Three different desalting procedures were assessed to remove salts and detergents from the rVWF samples prior to GEMMA analysis: membrane centrifugation (Nanosep™ 3K), size exclusion chromatography (MicroSpin™ G-25), and dialysis (Slide-A-Lyzer® 10K). Glycoprotein recovery after the desalting procedure was monitored with SDS-PAGE under reducing conditions. Figure 2 shows the SDS-PAGE data comparing rVWF sample A without desalting (lane 2) and desalted two times with Microspin™ G-25 (size exclusion chromatography) columns (lane 3). Untreated rVWF sample A shows two protein bands at approximately 269 kDa and 339 kDa, confirming the data achieved by CGE-on-the-chip. Size exclusion chromatography (MicroSpin™ G-25) of rVWF sample A also yielded two distinct protein bands, slightly fainter than the protein bands obtained from the untreated sample. Dialysis of rVWF samples resulted in extensive sample loss, most likely caused by adsorption of the highly adhesive rVWF to the dialysis membrane. There was little analyte lost when the sample was desalted using membrane centrifugation (Nanosep™ 3K).

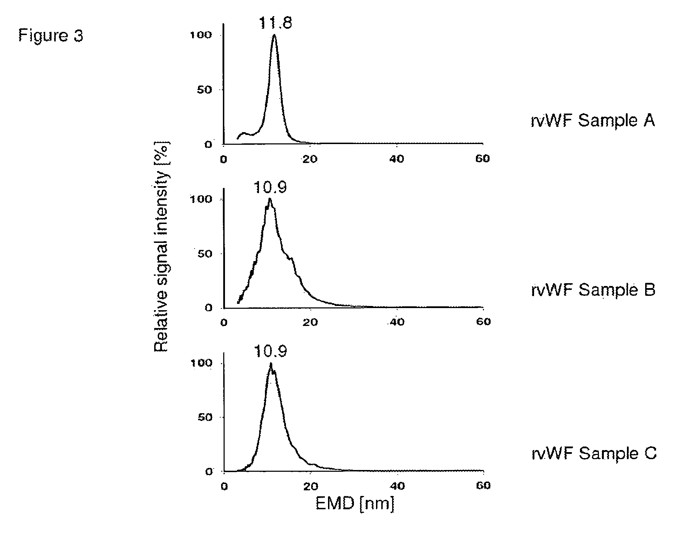

Figure 3 shows nano ES GEMMA spectra of reduced rVWF samples A to C having different rVWF concentrations. GEMMA spectra of rVWF sample A (Figure 3, top) shows an intense peak at 11.9 nm (± 1.6 %) corresponding to a molecular mass of 298.8 kDa, whereas rVWF sample B (Figure 3, center) and C (Figure 3, bottom) show peaks at 10.9 nm, corresponding to a molecular mass of 227 kDa (± 2.5 %).

The resolution of the nDMA used was not sufficient to clearly separate both molecular ions for the mature and pro-rVWF. In fact, the peak at 11.8 nm was the average value between 10.9 nm (measured from sample B and C) and 12.7 nm (the base for this value were MALDI TOF data). Samples B and C contain mostly mature and little pro-rVWF, which was confirmed by their nano ES GEMMA spectra. The concentration of the pro-rVWF was negligible, which corroborates the CGE-on-the-chip data.

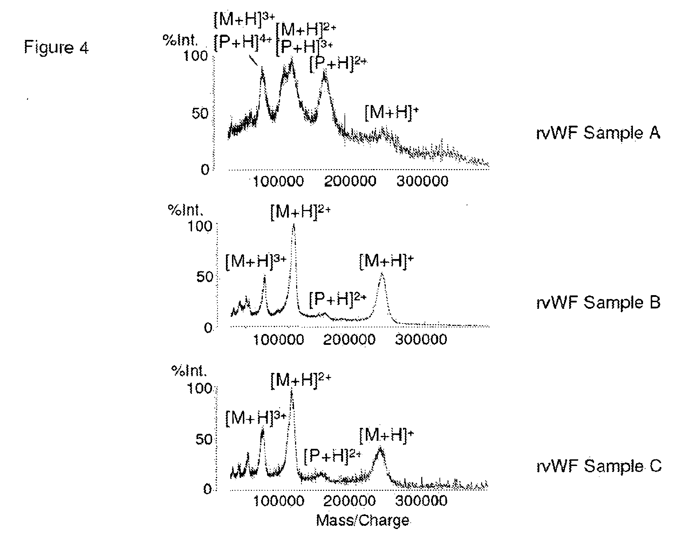

The rVWF samples were also analyzed by MALDI TOF MS. Due to its tolerance to salts and detergents, no extensive sample desalting/purification was needed. After reduction of disulfide bonds to obtain the rVWF monomer, salts and detergents were removed by a ZipTip^{™} desalting approach. Figure 4 shows the positive ion MALDI mass spectra of rVWF samples A to C after the desalting step. The MALDI-TOF spectra showed several ionized species of rVWF: [M + H]⁺ (*m*/*z* 256100), [M + 2H]²⁺ (*m*/*z* 127800) and [M + 3H]³⁺ (*m*/*z* 87600) ions of mature rVWF and [P + 2H]²⁺ (*m*/*z* 174400), [P + 3H]³⁺ (*m*/*z* 118100) and [P + 4H]⁴⁺ (*m*/*z* 87600) ions of pro-rVWF were detected in the positive ion mass spectrum of rVWF sample A (see Figure 4, top). A singly charged molecular ion of pro-rVWF was not detected.

Doubly charged mature rVWF (*m*/*z* 127800) and triply charged pro-rVWF species (*m*/*z* 118100) are not easily resolved or separated due to low MS resolution, resulting in a broad signal with two maxima. Separation of triply charged mature and quadruply charged pro-rVWF species was not observed, as the m/z values are indistinguishable (m/z 87000). MALDI mass spectra of rVWF sample B and C (Figure 4, center and bottom) containing mainly mature rVWF (confirmed by CGE-on-the-chip analysis, Figure 1) and small amounts of pro-rVWF, showed singly (m/z 249000), doubly (*m*/*z* 125600) and triply (*m*/*z* 85600) charged molecular ions of mature rVWF with excellent S/N ratios. These MALDI mass spectra also detected the doubly charged molecular ion of pro-rVWF (*m*/*z* 169300 sample B and *m*/*z* 167200 sample C) could be detected, in small amounts.

Table 1 summarizes molecular mass data obtained by nano ES GEMMA, MALDI TOF MS and CGE-on-the-chip. By means of external mass calibration of the MALDI TOF mass spectrometer, molecular mass determination of rVWF species was possible with a precision of ± 0.8 %. The experimental molecular mass values and calculated values based on the amino acid sequence of VWF^{38,3} were within a Δm of 27 to 43 kDa for pro-rVWF and a Δm of 30 to 34 kDa for mature rVWF. These Δm values can be attributed to the carbohydrate moiety of the rVWF, indicating a glycosylation degree of 8 to 12 % for pro-rVWF and 12 to 14 % for mature rVWF.

Separation of the mature and pro-rVWF in sample A was not observed for the nanoES GEMMA analysis. Due to the lower resolution, a mixture of both species was detected at 11.9 nm, corresponding to a molecular mass of 298.8 kDa (± 1.6 %). Back-calculation from the MALDI MS data, 256.1 kDa (mature rVWF) and 349.8 kDa (pro-rVWF) indicated an EMD of 11.3 nm and 12.6 nm, respectively. Samples B and C had only low amounts of pro-rVWF, and only mature rVWF species was detected in their nano ES GEMMA analyses, which corresponded to a molecular mass of 227.4 kDa (± 2.5 %).

**Table 1**

| Sample | Nano ES GEMMA | | MALDI TOF MS | | CGE-on-the-chip | |
|---|---|---|---|---|---|---|
| | Mature | Pro | Mature | Pro | Mature | Pro |
| A | 298.8¹ | | 256.1 | 349.8 | 277.8 | 341.9 |
| B | 227.4 | - | 249.1 | 338.6 | 277.2 | 342.5 |
| C | 227.4 | - | 248.9 | 334.6 | 276.5 | 341.3 |

External MS mass calibration provided information about the molecular mass of the rVWF, which compared to reported values. Reported values were about 226 kDa for mature and about 307 kDa for pro-rVWF and MALDI TOF MS values were 256.1 kDa (± 0.8 %) for mature rVWF and 349.8 kDa (± 0.8 %) for pro-rVWF. Mass differences of 30 to 34 kDa (mature rVWF) and 27 to 43 kDa were obtained, which can be related to the 22 N- and O-glycans of rVWF, indicating a glycosylation degree of 12 to 14 % for mature and of 8 to 12 % for pro-rVWF. Nano ES GEMMA analysis of rVWF sample A showed a size of 11.9, indicated a molecular mass of 298.8 kDa (±1.6 %), which was actually a mixture of the mature rVWF and pro-rVWF. Nano ES GEMMA analysis of rVWF samples B and C having low amounts of pro-rVWF resulted in detection of the mature rVWF with a molecular mass of 227.4 kDa (± 2.5 %), corresponding to a size of 10.9 nm.

### REFERENCES

1. Ruggeri ZM, Zimmerman TS. Blood 1987; 70: 895
2. Sadler JE. The Journal of Biological Chemistry 1991; 266: 22777
3. Dent JA, et al. Journal of Clinical Investigation 1991; 88: 774
4. Ruggeri ZM. Current Opinion in Hematology 2003; 10: 142
5. Furlan M. Annals of Hematology 1996; 72: 341
6. Jaffe EA, et al. Journal of Clinical Investigation 1973; 52: 2757
7. Wagner DD, et al. Journal of Cell Biology 1984; 99: 2123
8. Nachman R, et al. Journal of Clinical Investigation 1977; 60: 914
9. Titani K, et al. Biochemistry 1986; 25: 3171
10. Marti T, et al. Biochemistry 1987; 26: 8099
11. Wagner DD, et al. Blood 1987; 69: 27
12. Sodetz JM, et al. The Journal of Biological Chemistry 1979; 254: 10754
13. Matsui T, et al. The Journal of Biological Chemistry 1992; 267: 8723
14. Millar CM, et al. Blood Reviews 2006; 20: 83
15. Carew JA, et al. Blood 1990; 76: 2530
16. Dent JA, et al. Proceedings of the Notional Academy of Sciences of the United States of America 1990; 87: 6306
17. Carew JA, et al. Journal of Clinical Investigation 1992; 90: 2258
18. van Schooten CJ, et al. Blood 2007; 109: 2430
19. Wagner DD, et al. Journal. of Cell Biology 1982; 95: 355
20. Ruggeri ZM. Journal of Thrombosis and Haemostasis 2003; 1: 1335
21. Vlot AJ, et al. Thrombosis and Haemostasis 1998; 79: 456
22. Budde U, et al. Reviews in Clinical and Experimental Hematology 2001; 5: 335
23. Federici AB. Haemophilia 2008; 14 Suppl 1: 5
24. Menache D, et al. Thrombosis and Haemostasis 1997; 78: 566
25. Thompson AR. Haematologica Reports 2005; 1: 32
26. Mannuccio PM, et al. Blood 1994; 83: 3018
27. Fischer BE. Journal of Thrombosis and Thrombolysis 1999; 8: 197
28. Turecek PL, et al. Histochemistry and cell biology 2002; 117: 123
29. Rand JH, et al. British journal of haematology 1987; 67: 433
30. Fischer B, et al. FEBS Letters 1994; 351: 345
31. Kaufman SL, et al. Analytical Chemistry 1996; 68: 1895
32. Koropchak JA, et al. Analytical Chemistry 1999; 71: 386A
33. Kaddis CS, et al. Analytical Chemistry 2007; 79: 1778
34. Bacher G, et al. Journal of Mass Spectrometry 2001; 36: 1038
35. Muller R, et al. Rapid Communication in Mass Spectrometry 2006; 20: 3803
36. Karas M, et al. Analytical Chemistry 1988; 60: 2299
37. Laschober C, et al. Journal of Experimental Nanosciences 2007; 2: 291
38. http://www.expasy.org/uniprot/P04275 (May 2008)
39. Müller R, et al, Analytical, and Bioanalytical Chemistry 2007; 389: 1859

## Claims

1. A method for external and internal calibration of a MALDI mass spectrum for determining molecular weight of a high molecular weight analyte, comprising
a) obtaining a mass spectrum of a recombinant von Willebrand Factor (rVWF) of known molecular weight;
b) analyzing an analyte via mass spectrometry to provide a mass spectrum, wherein the analyte has a molecular weight of greater than 200 kDa; and
c) determining the molecular weight of the analyte by correlating the mass spectrum of the analyte to the mass spectrum of the rVWF.

2. The method of claim 1, wherein the analyte has a molecular weight of at least 1000 kDa.

3. The method of claim 2, wherein the analyte has a molecular weight of at least 1500 kDa.

4. The method of claim 1, wherein the analyte comprises an protein larger than 200 kDa.

5. The method of claim 1, wherein the analyte comprises an immunoglobulin, a fibrinogen, a fibronectin, or a factor XIII.

6. The method of claim 1, wherein the mass spectrometry comprises matrix-assisted laser desorption ionization (MALDI) or matrix-assisted laser desorption ionization -time of flight (MALDI-TOF).

7. The method of claim 1, further comprising desalting the analyte prior to analyzing.

8. The method of claim 1, further comprising calculating the rVWF molecular weight by determining the number of monomers of VWF (SEQ ID NO: 1) in the rVWF.

9. The method of claim 8, wherein the monomers of VWF are connected by disulfide bonds.

## Patentansprüche

1. Verfahren zur externen und internen Kalibrierung eines MALDI-Massenspektrums zur Bestimmung des Molekulargewichts eines Analyts hohen Molekulargewichts, umfassend
a) Erhalten eines Massenspektrums eines rekombinanten von-Willebrand-Faktors (rVWF) bekannten Molekulargewichts,
b) Analysieren eines Analyts durch Massenspektrometrie, um ein Massenspektrum bereitzustellen, wobei der Analyt ein Molekulargewicht von grö-βer als 200 kDa aufweist, und
c) Bestimmen des Molekulargewichts des Analyts durch Inbeziehungsetzen des Massenspektrums des Analyten mit dem Massenspektrum des rVWF.

2. Verfahren nach Anspruch 1, wobei der Analyt ein Molekulargewicht von mindestens 1000 kDa aufweist.

3. Verfahren nach Anspruch 2, wobei der Analyt ein Molekulargewicht von mindestens 1500 kDa aufweist.

4. Verfahren nach Anspruch 1, wobei der Analyt ein Protein, das größer als 200 kDa ist, umfasst.

5. Verfahren nach Anspruch 1, wobei der Analyt ein Immunglobulin, ein Fibrinogen, ein Fibronectin, oder einen Faktor XIII umfasst.

6. Verfahren nach Anspruch 1, wobei die Massenspektrometrie Matrix-unterstützte Laser-Desorptionlionisation (MALDI) oder Matrix-unterstützte Laser-Desorption/lonisation mit Flugzeitanalyse (MALDI-TOF) umfasst.

7. Verfahren nach Anspruch 1, weiter umfassend das Entsalzen des Analyts vor der Analyse.

8. Verfahren nach Anspruch 1, weiter umfassend das Berechnen des Molekulargewichts von rVWF durch Bestimmen der Anzahl von VWF-Monomeren (SEQ ID NO: 1) in dem rVWF.

9. Verfahren nach Anspruch 8, wobei die VWF-Monomere durch Disulfidbrücken verbunden sind.

## Revendications

1. Procédé d'étalonnage externe et interne d'un spectre de masse MALDI pour déterminer le poids moléculaire d'un analyte de poids moléculaire élevé, comprenant :
a) l'obtention d'un spectre de masse d'un facteur de von Willebrand recombinant (rVWF) de poids moléculaire connu ;
b) l'analyse d'un analyte par spectrométrie de masse pour fournir un spectre de masse, dans lequel l'analyte possède un poids moléculaire supérieur à 200 kDa ; et
c) la détermination du poids moléculaire de l'analyte en effectuant une corrélation entre le spectre de masse de l'analyte et le spectre de masse du rVWF.

2. Procédé selon la revendication 1, dans lequel l'analyte possède un poids moléculaire d'au moins 1000 kDa.

3. Procédé selon la revendication 2, dans lequel l'analyte possède un poids moléculaire d'au moins 1500 kDa.

4. Procédé selon la revendication 1, dans lequel l'analyte comprend une protéine de plus de 200 kDa.

5. Procédé selon la revendication 1, dans lequel l'analyte comprend une immunoglobuline, un fibrinogène, une fibronectine ou un facteur XIII.

6. Procédé selon la revendication 1, dans lequel la spectrométrie de masse comprend la spectrométrie de masse à désorption-ionisation laser assistée par matrice (MALDI) ou la spectrométrie de masse à désorption-ionisation laser assistée par matrice et à temps de vol (MALDI-TOF).

7. Procédé selon la revendication 1, comprenant en outre le dessalage de l'analyte avant son analyse.

8. Procédé selon la revendication 1, comprenant en outre le calcul du poids moléculaire du rVWF en déterminant le nombre de monomères de VWF (SEQ ID NO : 1) dans le rVWF.

9. Procédé selon la revendication 8, dans lequel les monomères de VWF sont reliés par des ponts disulfures.


### SEQUENCE LISTIPIG

<110> KEMPTNER, et al.
<120> RECOMBINANT VON WILLEBRAND FACTOR AS MOLECULAR WEIGHT MARKER FOR MASS SPECTROMETRY ANALYSES
<130> 31315/44044A
<140> PCTUS0968258
   <141> 2009-12-16
<150> US-61/140,475
   <151> 2008-12-23
<160> 1
<170> PatentIn version 3.5
<210> 1
   <211> 2050
   <212> PRT
   <213> Artificial Sequence
<220>
   <223> Synthetic Polypeptide
<220>
   <221> MISC_FEATURE
   <223> mature vWF
<400> 1

Ser Leu Ser Cys Arg Pro Pro Met Val Lys Leu Val Cys Pro Ala Asp
1               5                   10                  15

Asn Leu Arg Ala Glu Gly Leu Glu Cys Thr Lys Thr Cys Gln Asn Tyr
            20                  25                  30

Asp Leu Glu Cys Met Ser Met Gly Cys Val Ser Gly Cys Leu Cys Pro
        35                  40                  45

Pro Gly Met Val Arg His Glu Asn Arg Cys Val Ala Leu Glu Arg Cys
    50                  55                  60

Pro Cys Phe His Gln Gly Lys Glu Tyr Ala Pro Gly Glu Thr Val Lys
65                  70                  75                  80

Ile Gly Cys Asn Thr Cys Val Cys Arg Asp Arg Lys Trp Asn Cys Thr
                85                  90                  95

Asp His Val Cys Asp Ala Thr Cys Ser Thr Ile Gly Met Ala His Tyr
            100                 105                 110

Leu Thr Phe Asp Gly Leu Lys Tyr Leu Phe Pro Gly Glu Cys Gln Tyr
        115                 120                 125

Val Leu Val Gln Asp Tyr Cys Gly Ser Asn Pro Gly Thr Phe Arg Ile
    130                 135                 140

Leu Val Gly Asn Lys Gly Cys Ser His Pro Ser Val Lys Cys Lys Lys
145                 150                 155                 160

Arg Val Thr Ile Leu Val Glu Gly Gly Glu Ile Glu Leu Phe Asp Gly
                165                 170                 175

Glu Val Asn Val Lys Arg Pro Met Lys Asp Glu Thr His Phe Glu Val
            180                 185                 190

Val Glu Ser Gly Arg Tyr Ile Ile Leu Leu Leu Gly Lys Ala Leu Ser
        195                 200                 205

Val Val Trp Asp Arg His Leu Ser Ile Ser Val Val Leu Lys Gln Thr
    210                 215                 220

Tyr Gln Glu Lys Val Cys Gly Leu Cys Gly Asn Phe Asp Gly Ile Gln
225                 230                 235                 240

Asn Asn Asp Leu Thr Ser Ser Asn Leu Gln Val Glu Glu Asp Pro Val
                245                 250                 255

Asp Phe Gly Asn Ser Trp Lys Val Ser Ser Gln Cys Ala Asp Thr Arg
            260                 265                 270

Lys Val Pro Leu Asp Ser Ser Pro Ala Thr Cys His Asn Asn Ile Met
        275                 280                 285

Lys Gln Thr Met Val Asp Ser Ser Cys Arg Ile Leu Thr Ser Asp Val
    290                 295                 300

Phe Gln Asp Cys Asn Lys Leu Val Asp Pro Glu Pro Tyr Leu Asp Val
305                 310                 315                 320

Cys Ile Tyr Asp Thr Cys Ser Cys Glu Ser Ile Gly Asp Cys Ala Cys
                325                 330                 335

Phe Cys Asp Thr Ile Ala Ala Tyr Ala His Val Cys Ala Gln His Gly
            340                 345                 350

Lys Val Val Thr Trp Arg Thr Ala Thr Leu Cys Pro Gln Ser Cys Glu
        355                 360                 365

Glu Arg Asn Leu Arg Glu Asn Gly Tyr Glu Cys Glu Trp Arg Tyr Asn
    370                 375                 380

Ser Cys Ala Pro Ala Cys Gln Val Thr Cys Gln His Pro Glu Pro Leu
385                 390                 395                 400

Ala Cys Pro Val Gln Cys Val Glu Gly Cys His Ala His Cys Pro Pro
                405                 410                 415

Gly Lys Ile Leu Asp Glu Leu Leu Gln Thr Cys Val Asp Pro Glu Asp
            420                 425                 430

Cys Pro Val Cys Glu Val Ala Gly Arg Arg Phe Ala Ser Gly Lys Lys
        435                 440                 445

Val Thr Leu Asn Pro Ser Asp Pro Glu His Cys Gln Ile Cys His Cys
    450                 455                 460

Asp Val Val Asn Leu Thr Cys Glu Ala Cys Gln Glu Pro Gly Gly Leu
465                 470                 475                 480

Val Val Pro Pro Thr Asp Ala Pro Val Ser Pro Thr Thr Leu Tyr Val
                485                 490                 495

Glu Asp Ile Ser Glu Pro Pro Leu His Asp Phe Tyr Cys Ser Arg Leu
            500                 505                 510

Leu Asp Leu Val Phe Leu Leu Asp Gly Ser Ser Arg Leu Ser Glu Ala
        515                 520                 525

Glu Phe Glu Val Leu Lys Ala Phe Val Val Asp Met Met Glu Arg Leu
    530                 535                 540

Arg Ile Ser Gln Lys Trp Val Arg Val Ala Val Val Glu Tyr His Asp

545                 550                 555                 560

Gly Ser His Ala Tyr Ile Gly Leu Lys Asp Arg Lys Arg Pro Ser Glu
                565                 570                 575

Leu Arg Arg Ile Ala Ser Gln Val Lys Tyr Ala Gly Ser Gln Val Ala
            580                 585                 590

Ser Thr Ser Glu Val Leu Lys Tyr Thr Leu Phe Gln Ile Phe Ser Lys
        595                 600                 605

Ile Asp Arg Pro Glu Ala Ser Arg Ile Thr Leu Leu Leu Met Ala Ser
    610                 615                 620

Gln Glu Pro Gln Arg Met Ser Arg Asn Phe Val Arg Tyr Val Gln Gly
625                 630                 635                 640

Leu Lys Lys Lys Lys Val Ile Val Ile Pro Val Gly Ile Gly Pro His
                645                 650                 655

Ala Asn Leu Lys Gln Ile Arg Leu Ile Glu Lys Gln Ala Pro Glu Asn
            660                 665                 670

Lys Ala Phe Val Leu Ser Ser Val Asp Glu Leu Glu Gln Gln Arg Asp
        675                 680                 685

Glu Ile Val Ser Tyr Leu Cys Asp Leu Ala Pro Glu Ala Pro Pro Pro
    690                 695                 700

Thr Leu Pro Pro Asp Met Ala Gln Val Thr Val Gly Pro Gly Leu Leu
705                 710                 715                 720

Gly Val Ser Thr Leu Gly Pro Lys Arg Asn Ser Met Val Leu Asp Val
                725                 730                 735

Ala Phe Val Leu Glu Gly Ser Asp Lys Ile Gly Glu Ala Asp Phe Asn
            740                 745                 750

Arg Ser Lys Glu Phe Met Glu Glu Val Ile Gln Arg Met Asp Val Gly
        755                 760                 765

Gln Asp Ser Ile His Val Thr Val Leu Gln Tyr Ser Tyr Met Val Thr
    770                 775                 780

Val Glu Tyr Pro Phe Ser Glu Ala Gln Ser Lys Gly Asp Ile Leu Gln
785                 790                 795                 800

Arg Val Arg Glu Ile Arg Tyr Gln Gly Gly Asn Arg Thr Asn Thr Gly
                805                 810                 815

Leu Ala Leu Arg Tyr Leu Ser Asp His Ser Phe Leu Val Ser Gln Gly
            820                 825                 830

Asp Arg Glu Gln Ala Pro Asn Leu Val Tyr Met Val Thr Gly Asn Pro
        835                 840                 845

Ala Ser Asp Glu Ile Lys Arg Leu Pro Gly Asp Ile Gln Val Val Pro
    850                 855                 860

Ile Gly Val Gly Pro Asn Ala Asn Val Gln Glu Leu Glu Arg Ile Gly
865                 870                 875                 880

Trp Pro Asn Ala Pro Ile Leu Ile Gln Asp Phe Glu Thr Leu Pro Arg
                885                 890                 895

Glu Ala Pro Asp Leu Val Leu Gln Arg Cys Cys Ser Gly Glu Gly Leu
            900                 905                 910

Gln Ile Pro Thr Leu Ser Pro Ala Pro Asp Cys Ser Gln Pro Leu Asp
        915                 920                 925

Val Ile Leu Leu Leu Asp Gly Ser Ser Ser Phe Pro Ala Ser Tyr Phe
    930                 935                 940

Asp Glu Met Lys Ser Phe Ala Lys Ala Phe Ile Ser Lys Ala Asn Ile
945                 950                 955                 960

Gly Pro Arg Leu Thr Gln Val Ser Val Leu Gln Tyr Gly Ser Ile Thr
                965                 970                 975

Thr Ile Asp Val Pro Trp Asn Val Val Pro Glu Lys Ala His Leu Leu
            980                 985                 990

Ser Leu Val Asp Val Met Gln Arg Glu Gly Gly Pro Ser Gln Ile Gly
        995                 1000                1005

Asp Ala Leu Gly Phe Ala Val Arg Tyr Leu Thr Ser Glu Met His
    1010                1015                1020

Gly Ala Arg Pro Gly Ala Ser Lys Ala Val Val Ile Leu Val Thr
    1025                1030                1035

Asp Val Ser Val Asp Ser Val Asp Ala Ala Ala Asp Ala Ala Arg
    1040                1045                1050

Ser Asn Arg Val Thr Val Phe Pro Ile Gly Ile Gly Asp Arg Tyr
    1055                1060                1065

Asp Ala Ala Gln Leu Arg Ile Leu Ala Gly Pro Ala Gly Asp Ser
    1070                1075                1080

Asn Val Val Lys Leu Gln Arg Ile Glu Asp Leu Pro Thr Met Val
    1085                1090                1095

Thr Leu Gly Asn Ser Phe Leu His Lys Leu Cys Ser Gly Phe Val
    1100                1105                1110

Arg Ile Cys Met Asp Glu Asp Gly Asn Glu Lys Arg Pro Gly Asp
    1115                1120                1125

Val Trp Thr Leu Pro Asp Gln Cys His Thr Val Thr Cys Gln Pro
    1130                1135                1140

Asp Gly Gln Thr Leu Leu Lys Ser His Arg Val Asn Cys Asp Arg
    1145                1150                1155

Gly Leu Arg Pro Ser Cys Pro Asn Ser Gln Ser Pro Val Lys Val
    1160                1165                1170

Glu Glu Thr Cys Gly Cys Arg Trp Thr Cys Pro Cys Val Cys Thr
    1175                1180                1185

Gly Ser Ser Thr Arg His Ile Val Thr Phe Asp Gly Gln Asn Phe
    1190                1195                1200

Lys Leu Thr Gly Ser Cys Ser Tyr Val Leu Phe Gln Asn Lys Glu
    1205                1210                1215

Gln Asp Leu Glu Val Ile Leu His Asn Gly Ala Cys Ser Pro Gly
    1220                1225                1230

Ala Arg Gln Gly Cys Met Lys Ser Ile Glu Val Lys His Ser Ala
    1235                1240                1245

Leu Ser Val Glu Leu His Ser Asp Met Glu Val Thr Val Asn Gly
    1250                1255                1260

Arg Leu Val Ser Val Pro Tyr Val Gly Gly Asn Met Glu Val Asn
    1265                1270                1275

Val Tyr Gly Ala Ile Met His Glu Val Arg Phe Asn His Leu Gly
    1280                1285                1290

His Ile Phe Thr Phe Thr Pro Gln Asn Asn Glu Phe Gln Leu Gln
    1295                1300                1305

Leu Ser Pro Lys Thr Phe Ala Ser Lys Thr Tyr Gly Leu Cys Gly
    1310                1315                1320

Ile Cys Asp Glu Asn Gly Ala Asn Asp Phe Met Leu Arg Asp Gly
    1325                1330                1335

Thr Val Thr Thr Asp Trp Lys Thr Leu Val Gln Glu Trp Thr Val
    1340                1345                1350

Gln Arg Pro Gly Gln Thr Cys Gln Pro Ile Leu Glu Glu Gln Cys
    1355                1360                1365

Leu Val Pro Asp Ser Ser His Cys Gln Val Leu Leu Leu Pro Leu
    1370                1375                1380

Phe Ala Glu Cys His Lys Val Leu Ala Pro Ala Thr Phe Tyr Ala
    1385                1390                1395

Ile Cys Gln Gln Asp Ser Cys His Gln Glu Gln Val Cys Glu Val
    1400                1405                1410

Ile Ala Ser Tyr Ala His Leu Cys Arg Thr Asn Gly Val Cys Val
    1415                1420                1425

Asp Trp Arg Thr Pro Asp Phe Cys Ala Met Ser Cys Pro Pro Ser

1430                    1435                    1440

Leu Val Tyr Asn His Cys Glu His Gly Cys Pro Arg His Cys Asp
    1445                    1450                    1455

Gly Asn Val Ser Ser Cys Gly Asp His Pro Ser Glu Gly Cys Phe
    1460                    1465                    1470

Cys Pro Pro Asp Lys Val Met Leu Glu Gly Ser Cys Val Pro Glu
    1475                    1480                    1485

Glu Ala Cys Thr Gln Cys Ile Gly Glu Asp Gly Val Gln His Gln
    1490                    1495                    1500

Phe Leu Glu Ala Trp Val Pro Asp His Gln Pro Cys Gln Ile Cys
    1505                    1510                    1515

Thr Cys Leu Ser Gly Arg Lys Val Asn Cys Thr Thr Gln Pro Cys
    1520                    1525                    1530

Pro Thr Ala Lys Ala Pro Thr Cys Gly Leu Cys Glu Val Ala Arg
    1535                    1540                    1545

Leu Arg Gln Asn Ala Asp Gln Cys Cys Pro Glu Tyr Glu Cys Val
    1550                    1555                    1560

Cys Asp Pro Val Ser Cys Asp Leu Pro Pro Val Pro His Cys Glu
    1565                    1570                    1575

Arg Gly Leu Gln Pro Thr Leu Thr Asn Pro Gly Glu Cys Arg Pro
    1580                    1585                    1590

Asn Phe Thr Cys Ala Cys Arg Lys Glu Glu Cys Lys Arg Val Ser
    1595                    1600                    1605

Pro Pro Ser Cys Pro Pro His Arg Leu Pro Thr Leu Arg Lys Thr
    1610                    1615                    1620

Gln Cys Cys Asp Glu Tyr Glu Cys Ala Cys Asn Cys Val Asn Ser
    1625                    1630                    1635

Thr Val Ser Cys Pro Leu Gly Tyr Leu Ala Ser Thr Ala Thr Asn
    1640                    1645                    1650

Asp Cys Gly Cys Thr Thr Thr Thr Cys Leu Pro Asp Lys Val Cys
    1655                1660                1665

Val His Arg Ser Thr Ile Tyr Pro Val Gly Gln Phe Trp Glu Glu
    1670                1675                1680

Gly Cys Asp Val Cys Thr Cys Thr Asp Met Glu Asp Ala Val Met
    1685                1690                1695

Gly Leu Arg Val Ala Gln Cys Ser Gln Lys Pro Cys Glu Asp Ser
    1700                1705                1710

Cys Arg Ser Gly Phe Thr Tyr Val Leu His Glu Gly Glu Cys Cys
    1715                1720                1725

Gly Arg Cys Leu Pro Ser Ala Cys Glu Val Val Thr Gly Ser Pro
    1730                1735                1740

Arg Gly Asp Ser Gln Ser Ser Trp Lys Ser Val Gly Ser Gln Trp
    1745                1750                1755

Ala Ser Pro Glu Asn Pro Cys Leu Ile Asn Glu Cys Val Arg Val
    1760                1765                1770

Lys Glu Glu Val Phe Ile Gln Gln Arg Asn Val Ser Cys Pro Gln
    1775                1780                1785

Leu Glu Val Pro Val Cys Pro Ser Gly Phe Gln Leu Ser Cys Lys
    1790                1795                1800

Thr Ser Ala Cys Cys Pro Ser Cys Arg Cys Glu Arg Met Glu Ala
    1805                1810                1815

Cys Met Leu Asn Gly Thr Val Ile Gly Pro Gly Lys Thr Val Met
    1820                1825                1830

Ile Asp Val Cys Thr Thr Cys Arg Cys Met Val Gln Val Gly Val
    1835                1840                1845

Ile Ser Gly Phe Lys Leu Glu Cys Arg Lys Thr Thr Cys Asn Pro
    1850                1855                1860

Cys Pro Leu Gly Tyr Lys Glu Glu Asn Asn Thr Gly Glu Cys Cys
    1865                1870                1875

Gly Arg Cys Leu Pro Thr Ala Cys Thr Ile Gln Leu Arg Gly Gly
    1880                1885                1890

Gln Ile Met Thr Leu Lys Arg Asp Glu Thr Leu Gln Asp Gly Cys
    1895                1900                1905

Asp Thr His Phe Cys Lys Val Asn Glu Arg Gly Glu Tyr Phe Trp
    1910                1915                1920

Glu Lys Arg Val Thr Gly Cys Pro Pro Phe Asp Glu His Lys Cys
    1925                1930                1935

Leu Ala Glu Gly Gly Lys Ile Met Lys Ile Pro Gly Thr Cys Cys
    1940                1945                1950

Asp Thr Cys Glu Glu Pro Glu Cys Asn Asp Ile Thr Ala Arg Leu
    1955                1960                1965

Gln Tyr Val Lys Val Gly Ser Cys Lys Ser Glu Val Glu Val Asp
    1970                197